# EUROPEAN PATENT APPLICATION

(11) **EP 2 243 431 A1**
(43) Date of publication of application: **27.10.2010**
(21) Application number: 09709197.9
(22) Date of filing: 26.01.2009
(51) Int. Cl.: A61B 8/08

(54) **ULTRASONOGRAPHIC DEVICE**

(30) Priority: 07.02.2008 JP 2008028191
(71) Applicant: Hitachi Medical Corporation, Chiyoda-ku Tokyo 101-0021 (JP)
(72) Inventor: WAKI, Koji, Tokyo 101-0021 (JP)
(74) Representative: MERH-IP Matias Erny Reichl Hoffmann
(86) International application number: PCT/JP2009/051195
(87) International publication number: WO 2009/098961

(57) **Abstract**

An ultrasonic diagnostic apparatus that can display various information obtained by acquiring an elasticity image in association with the elasticity image or a biometric simulation image is implemented. The ultrasonic diagnostic apparatus 1 of this invention generates an elasticity image representing hardness or softness of a tissue at a cross-sectional site on the basis of a pair of RF signal frame data which are obtained by transmitting/receiving ultrasonic wave to/from an object 10 and different in acquisition time, and also generates a biometric simulation image 85 simulating the object. Display control means displays on a display unit an elasticity mark 101 gradated in accordance with hardness or softness of a tissue of interest (tumor) in a set area of an acquired elasticity image on a real-time basis or after the elasticity mark 101 is temporarily recorded in a memory while superimposing the elasticity mark 101 at a position on the biometric simulation image 85 at which the elasticity image is acquired.

## Description

### Technical Field

The present invention relates to an ultrasonic diagnostic apparatus, and specifically to an ultrasonic diagnostic apparatus for displaying on a display unit various information which are obtained by picking up an elasticity image representing hardness or softness of a tissue of an object.

### Background Art

An ultrasonic diagnostic apparatus is used to transmit an ultrasonic wave into an object through an ultrasonic probe, receive a reflection echo signal of the ultrasonic wave corresponding to the structure of a biomedical tissue from the inside of the object and generate an image such as an ultrasonic tomographic image or the like for diagnosis, for example.

It has been recently executed to acquire RF signal frame data while an object is pressed through an ultrasonic probe by a manual or mechanical method, a displacement of each part of a living body occurring due to the press is determined on the basis of a pair of RF signal frame data which are different in acquisition time and an elasticity image representing hardness or softness of a biomedical tissue is generated or acquired on the basis of the displacement data as described in Patent Document 1.

It is described in a cited document 2 that a tomographic image of an object is displayed and a probe mark representing a scan position of a probe is displayed and superimposed at a position on an illustration (body mark) image simulating a living body at which the tomographic image is acquired. If the tomographic image and the biometric simulation image on which the probe mark is superimposed are recorded in association with each other, the tomographic image and the examination site at which the tomographic image is acquired can be easily associated with each other after ultrasonic diagnosis.
Patent Document 1: JP-A-2000-060853
Patent document 2: JP-A-2007-202829

### Disclosure of the Invention

### Problem to be Solved by the Invention

However, the technique described in the patent document 2 takes it into no consideration that various information obtained by acquiring an elasticity image is displayed in association with an image such as the elasticity image, a biometric simulation image or the like.

That is, in a process of acquiring an elasticity image, information useful to diagnosis such as information representing a condition under which the elasticity image concerned is acquired, etc. can be obtained in addition to elasticity information representing hardness or softness of a tissue. Therefore, such information is displayed while properly associated with an image such as the elasticity image, or a biometric simulation image, whereby these information may be effectively and practically used for diagnosis.

Therefore, the present invention has an object to implement an ultrasonic diagnostic apparatus that can display various information obtained by acquiring an elasticity image while the information concerned is associated with the elasticity image or a biometric simulation image.

### Means of solving the Problem

In order to attain the above object, an ultrasonic diagnostic apparatus according to the present invention is characterized by comprising: an ultrasonic probe that transmits/receives an ultrasonic wave from/to an object; phasing and summing means that generates RF signal frame data of a cross-sectional site of the object on the basis of a reflection echo signal measured by the ultrasonic probe; elasticity image constructing means that generates an elasticity image representing hardness or softness of a tissue of the cross-sectional site on the basis of a pair of RF signal frame data acquired at different times; cross-sectional site information generating means that generates cross-sectional site information containing position information of the cross-sectional site at which the elasticity image is measured; and display control means that controls to display on a display unit the elasticity image generated by the elasticity image constructing means and the cross-sectional site information generated by the cross-sectional site information generating means while the elasticity image and the cross-sectional site information are associated with each other.

As described above, by displaying the elasticity image and the cross-sectional site information in association with each other, an examiner or the like who picks up an ultrasonic wave image can grasp a cross-sectional site of an object at which the elasticity image concerned is generated.

Furthermore, the cross-sectional site information contains gradation information based on hardness or softness of a tissue, and thus the gradation information on hardness or softness in a set area of the elasticity image can be grasped. Accordingly, it may be effectively and practically used for diagnosis.

Furthermore, it is preferable that biometric simulation image generating means for generating a biometric simulation image which simulates the object is provided and the display control means is configured to display the biometric simulation image while superimposing the cross-sectional site information on the biometric simulation image.

Still furthermore, it is preferable that the cross-sectional site information is set to a simulation mark as at least one of an elasticity image acquiring mark representing a cross-sectional site at which an elasticity image is acquired, and an elasticity mark which is gradated in accordance with hardness or softness of a tissue in a set area of the acquired elasticity image, and the simulation mark is superimposed on the biometric simulation image and displayed on the display unit on a real-time basis or after temporarily recorded in a memory.

That is, an examiner who picks up an ultrasonic image, a diagnosing person who checks a temporarily-recorded image to make a diagnosis, etc. can visually check through the biometric simulation image by the superimposed display of the elasticity image acquiring mark where an elasticity image is acquired in the object. Furthermore, by the superimposed display of the elasticity mark, elasticity information of a set area in the cross-sectional site at which the elasticity image is acquired, that is, a tissue of interest can be simply grasped through the biometric simulation image.

Furthermore, for example, if an examiner picks up all elasticity images of an object and a biometric simulation image having elasticity marks superimposed thereon is recorded, it can be easily determined which site of the object should be examined minutely when a diagnosing person checks the biometric simulation image. That is, the elasticity marks are gradated with hue, numerical values or the like, and thus a site at which a diseased tissue may exist because it is relatively hard can be grasped on the biometric simulation image at a glance. Then, ultrasonic images are picked up again to make a minute examination as occasion demands, thereby making a diagnosis and determining a treatment policy.

The display control means of the ultrasonic diagnostic apparatus according to the present invention may be configured so that a simulation mark is successively displayed so as to be superimposed on a biometric simulation image every time an elasticity image is acquired while simulation marks superimposed in the past are left on the biometric simulation image. For example, a simulation mark is superimposedly displayed with all simulation marks being left every time an elasticity image is acquired during a process of picking up ultrasonic images by the examiner, whereby a site at which the image is acquired can be grasped at a glance. Therefore, a desired image can be prevented from being omitted.

Furthermore, it is desired that an elasticity image corresponding to a simulation mark superimposed on a biometric simulation image is recorded in a memory in association with the simulation mark, and also when a simulation mark is selected through an interface, the display control means displays the elasticity image associated with the selected simulation mark.

Likewise, when an elasticity image corresponding to a simulation mark is acquired, an output transition detected by a pressure sensor provided to a contact face of the ultrasonic probe to the object may be recorded in a memory in association with the simulation mark concerned, and when a simulation mark is selected through an interface, at least one of the output transition of the pressure sensor associated with the selected simulation mark, an average amplitude of pressure determined from the output transition of the pressure sensor and an average value of pressure determined from the output transition of the pressure sensor may be displayed.

Accordingly, the diagnosing person selects a simulation mark on a biometric simulation image of interest while checking the biometric simulation image temporarily recorded in the memory, whereby an elasticity image at the cross-sectional site can be displayed or a pressure condition under which the elasticity image is acquired can be displayed. As described above, by supplying the diagnosing person with multiple information, the diagnosing person can check elasticity images to obtain more detailed information or acquire an elasticity image with excellent reproducibility again in consideration of the displayed pressure condition, so that this invention can be effectively and practically used for diagnosis.

### Effect of the Invention

According to the present invention, there can be implemented an ultrasonic diagnostic apparatus that can display various information obtained by acquiring an elasticity image while associating the information concerned with the elasticity image or a biometric simulation image.

### Brief Description of the Invention

[Fig. 1] Fig. 1 is a block diagram showing the overall construction of an ultrasonic diagnostic apparatus according to an embodiment.
[Fig. 2] Fig. 2 is a diagram showing a first embodiment of tissue displacement detection.
[Fig. 3] Fig. 3 is a diagram showing a second embodiment of tissue displacement detection.
[Fig. 4] Fig. 4 is a diagram showing a third embodiment of tissue displacement detection.
[Fig. 5] Fig. 5 is a diagram showing a fourth embodiment of tissue displacement detection.
[Fig. 6] Fig. 6 is a diagram showing a fifth embodiment of tissue displacement detection.
[Fig. 7] Fig. 7 is a flowchart showing the whole operation of the ultrasonic diagnostic apparatus and an operation of generating and displaying an elasticity image acquiring mark while superimposing the elasticity image acquiring mark on a biometric simulation image.
[Fig. 8] Fig. 8 is a diagram showing a first embodiment of display.
[Fig. 9] Fig. 9 is a diagram showing a second embodiment of display.
[Fig. 10] Fig. 10 is a flowchart showing the whole operation of the ultrasonic diagnostic apparatus and an operation of generating and displaying an elasticity mark while superimposing the elasticity mark on a biometric simulation image.
[Fig. 11] Fig. 11 is a diagram showing a third embodiment of display.
[Fig. 12] Fig. 12 is a diagram showing a detailed elasticity mark generating method.
[Fig. 13] Fig. 13 is a diagram showing a fourth embodiment of display.

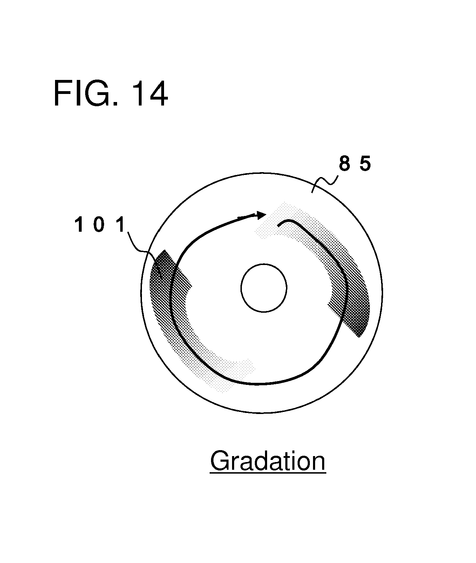

[Fig. 14] Fig. 14 is a diagram showing a fifth embodiment of display.
[Fig. 15] Fig. 15 is a diagram showing a sixth embodiment of display.
[Fig. 16] Fig. 16 is a diagram showing a seventh embodiment of display.
[Fig. 17] Fig. 17 is a diagram showing an eighth embodiment of display.
[Fig. 18] Fig. 18 is a diagram showing a ninth embodiment of display.

### Description of Reference Numerals

1 ultrasonic diagnostic apparatus, 10 object, 12 ultrasonic probe, 18 phasing and summing unit, 20 tomographic image constructing unit, 28 RF frame data selecting unit, 30 displacement measuring unit, 32 elasticity information calculating unit, 34 elasticity image constructing unit, 42 interface unit, 48 position information sensor, 50 coordinate calculating unit, 51 tissue displacement detecting unit, 52 mark generating unit, 54 biometric simulation image constructing unit, 56 memory, 65, 66 chasing point, 67 measured value of displacement, 70, 71 RF signal frame data, 73 measured value of pressure, 74 tissue displacement detecting switch, 85 biometric simulation image, 86 elasticity image acquiring mark, 87 pressure state display, 101 elasticity mark, 102 elasticity scale bar, 150 press mark, 160 blood flow mark, 161 blood flow scale bar

### Best Mode for Carrying Out the Invention

An embodiment of an ultrasonic diagnostic apparatus to which the present invention is applied will be described. In the following description, parts having the same functions are represented by the same reference numerals, and duplicative description thereof is omitted.

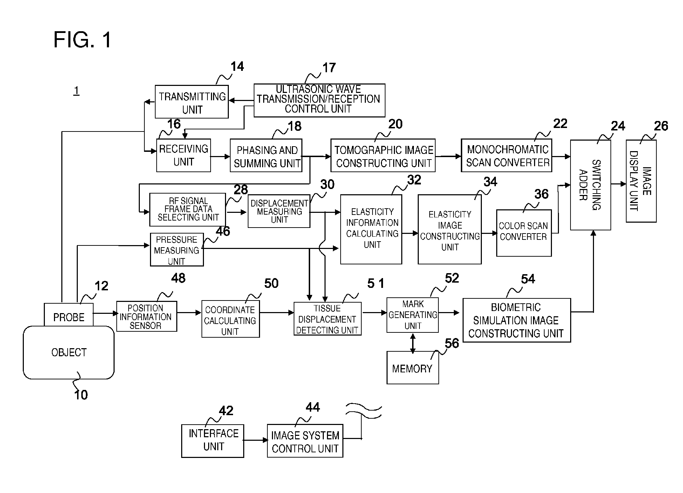

Fig. 1 is a block diagram showing the overall construction of an ultrasonic diagnostic apparatus according to the embodiment. As shown in Fig. 1, an ultrasonic diagnostic apparatus 1 is equipped with an ultrasonic probe 12 which is used in contact with an object 10, a transmitting unit 14 for repetitively transmitting ultrasonic waves to the object 10 through the ultrasonic probe 12 at a time interval, a receiving unit 16 for receiving time-series reflection echo signals occurring from the object 10, a transmission/reception control unit 17 for controlling the transmitting unit 14 and the receiving unit 16, and a phasing and summing unit 18 for phasing and summing reflection echoes received by the receiving unit 16.

Furthermore, there are provided a tomographic image constructing unit 20 for constructing a grayscale tomographic image such as a monochromatic tomographic image of the object on the basis of RF signal frame data from the phasing and summing unit 18, and a monochromatic scan converter 22 for converting an output signal of the tomographic image constructing unit 20 so that the converted output signal is matched with a display of an image display unit 26.

There are further provided an RF frame data selecting unit 28 for storing the RF signal frame data output from the phasing and summing unit 18 and selecting at least two frame data, a displacement measuring unit 30 for measuring a displacement of a biometric tissue of the object 10, an elasticity information calculating unit 32 for determining distortion or elasticity modulus from displacement information measured by the displacement measuring unit 30, an elasticity image constructing unit 34 for constructing a color elasticity image from the distortion or the elasticity modulus calculated by the elasticity information calculating unit 32, and a color scan converter 36 for converting an output signal of the elasticity image constructing unit 34 so that the converted output signal is matched with display of the image display unit 26.

There are further provided an image control unit 44 for outputting various kinds of control signals to the respective parts constituting the ultrasonic diagnostic apparatus, and an interface unit 42 for receiving an instruction from an examiner and outputs the instruction to the image control unit 44.

Here, the ultrasonic diagnostic apparatus 1 will be described in detail. The ultrasonic probe 12 is formed by arranging plural transducers, and it has a function of transmitting/receiving ultrasonic waves through the transducers to/from the object 10. The transmitting unit 14 has a function of driving the ultrasonic probe 12 to generate a wave-transmission pulse for generating an ultrasonic wave and also setting a convergence point of the ultrasonic wave to be transmitted to some depth. The receiving unit 16 amplifies the reflection echo signal received by the ultrasonic probe 12 with a predetermined gain to generate an RF signal, that is, a wave-reception signal. The phasing and summing unit 18 receives the RF signal amplified by the receiving unit 16 to perform phase control and forms an ultrasonic beam for one or plural convergence point(s), thereby generating the RF signal frame data.

The tomographic image constructing unit 20 receives the RF signal frame data from the phasing and summing unit 18 and executes signal processing such as gain correction, log compression, wave detection, edge enhancement, and filter processing to obtain tomographic image data. Furthermore, the monochromatic scan converter 22 has an A/D converter for converting tomographic image data from the tomographic image constructing unit 20 to a digital signal, a frame memory for storing plural converted tomographic image data in time-series, and a control control unit. The monochromatic scan converter 22 obtains in-object cross-sectional frame data stored in the frame memory as one image, and reads out the obtained cross-sectional frame data synchronously with TV.

The RF frame data selecting unit 28 stores plural RF signal frame data from the phasing and summing unit 18 and selects a pair of, that is, two RF signal frame data from the stored RF signal frame data group. For example, the RF signal frame data generated in time-series, that is, on the basis of an image frame rate from the phasing and summing unit 18 are successively stored into the RF frame data selecting unit 28, the stored RF signal frame data (N) is selected as first data and at the same time one RF signal frame data (X) is selected from RF signal frame data group (N-1, N-2, N-3, ..., N-M) which were stored in the past. Here, N, M and X represent index numbers affixed to the RF signal frame data, and set to natural numbers.

The displacement measuring unit 30 executes one-dimensional or two-dimensional correlation processing on the selected one pair of data, that is, the RF signal frame data (N) and the RF signal frame data (X) to determine a one-dimensional or two-dimensional displacement distribution concerning the displacement and moving vector, that is, the direction and magnitude of the displacement of a biometric tissue which corresponds to each point of a tomographic image. Here, a block matching method is used to detect the moving vector. The block matching method is defined as the processing of dividing an image into blocks each of which comprises NXNpixels, noting a block in an area of interest, searching from a previous frame a block which is most approximate to the block being noted, and refers to the searched block to determine a sample value through predictive coding, that is, differential calculation.

The elasticity information calculating unit 32 calculates the distortion and the elasticity modulus of a biometric tissue corresponding to each point on a tomographic image from a measured value output from the displacement measuring unit 30, for example, a moving vector and a pressure value output from a pressure measuring unit 46, and generates an elasticity image signal, that is, elasticity frame data on the basis of the distortion and the elasticity modulus.

At this time, the data of the distortion are calculated by spatially differentiating a moving amount, for example, a displacement of the biometric tissue. The data of the elasticity modulus is calculated by dividing the variation of pressure by the variation of distortion. For example, when the displacement measured by the displacement measuring unit 30 is represented by L(X) and the pressure measured by the pressure measuring unit 46 is represented by P(X), the distortion ΔS(X) can be calculated by spatially differentiating L(X), and thus it can be determined by using the mathematical expression of ΔS(X) = ΔL(X)/ΔX. Furthermore, Young's modulus Ym(X) of the elasticity modulus data is calculated according to the mathematical expression of Ym = (ΔP(X)) /ΔS(X). The elasticity modulus of a biometric tissue corresponding to each point of a tomographic image is determined from the Young's modulus Ym, and thus two-dimensional elasticity image data can be continuously obtained. The Young's modulus represents the ratio of simple tension stress applied to an object to strain occurring in parallel to the tension.

The elasticity image constructing unit 34 is configured to contain a frame memory and an image processor, and it secures elasticity frame data output in time-series from the elasticity information calculating unit 32 into the frame memory and executes image processing on the thus-secured frame data.

The color scan converter 36 has a function of adding hue information to the elasticity frame data from the elasticity image constructing unit 34. That is, it performs conversion to three primary colors of light, that is, red(R), green(G) and blue(B) on the basis of the elasticity frame data. For example, elasticity data having large distortion is converted to a red color code and elasticity data having small distortion is converted to a blue color code.

A switching adder 24 is configured to have a frame memory, an image processor and an image selecting unit. The frame memory stores tomographic image data from the monochromatic scan converter 22, elasticity image data from the color scan converter 36, and image data output from a biometric simulation image control unit 44 described later. Furthermore, the image processor has a function of combining the tomographic image data and the elasticity image data secured in the frame memory with changing the composite rate. Brightness information and hue information of each pixel of a composite image are obtained by adding respective information of a monochromatic tomographic image and a color elasticity image in the composite rate. Furthermore, the image selecting unit selects an image to be displayed on the image display unit 26 from the tomographic image data and the elasticity image data in the frame memory, composite image data of the image processor and biometric simulation image data.

Next, a feature portion of the ultrasonic diagnostic apparatus according to this embodiment will be described. The probe 12 is provided with a position information sensor 48 such as a magnetic sensor, an angular velocity sensor, or an infrared sensor, for example. Furthermore, there are also provided a coordinate calculating unit 50 for calculating a three-dimensional coordinate position of the probe on the basis of outputs of the position information sensor 48, a tissue displacement detecting unit 51 for detecting that the tissue of the cross-sectional site of the object is displaced and a mark generating unit 52 for generating an elasticity image acquiring mark, an elasticity mark or the like on the basis of an output of the coordinate calculating unit 50 and an output of the tissue displacement detecting unit 51.

Furthermore, there are also provided a biometric simulation image constructing unit 54 for generating a biometric simulation image such as an illustration, or a photograph which illustrates the object and superimposing a simulation mark generated in the mark generating unit 52 such as the elasticity image acquiring mark or the elasticity mark gradated in accordance with hardness or softness of a tissue in a set area of the elasticity image at a position on the biometric simulation image at which the elasticity image is acquired, and a memory 56 for storing information such as position information, and elasticity information of the cross-sectional site of the object at which the elasticity image is acquired.

Next, each embodiment of the tissue displacement detecting unit 51 for detecting that the tissue of the cross-sectional site of the object is displaced will be described.

### (First Embodiment of Tissue Displacement Detection)

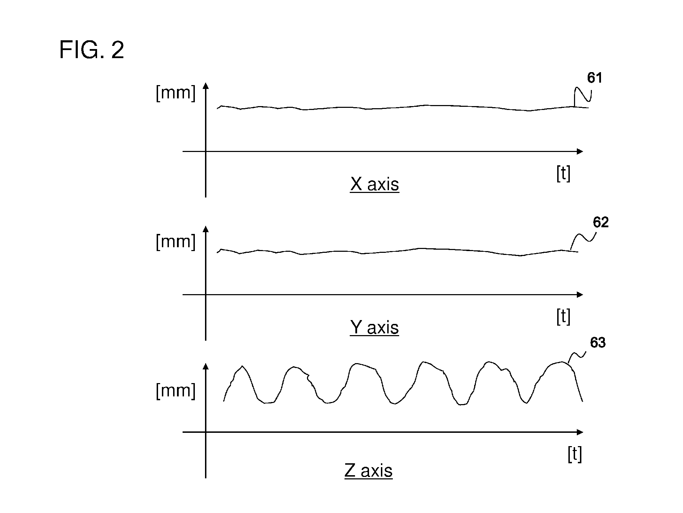

This embodiment is applied to a case where a pressing/releasing operation is executed on an object by using a probe to generate an elasticity image. For example, when an elasticity image is generated while the press/release operation is repetitively executed so as to induce a minute distortion variation from a state that the probe is pressed against the object to apply a fixed stress to the object, a signal detected by the position information sensor 48 mounted on the probe is input to the coordinate calculating unit 50, and divisionally processed on three-dimensional coordinate axes of X-axis, Y-axis and Z-axis as shown in Fig. 2 in the coordinate calculating unit 50. Fig. 2 is a diagram showing an example of variation of the coordinate position of the probe on each coordinate axis of the X-axis, the Y-axis and the Z-axis of the three-dimensional coordinate of the probe, which is output from the coordinate calculating unit 50.

In this embodiment, a coordinate 61 of the X-axis and a coordinate 62 of the Y-axis are substantially fixed and thus they are not displaced. A coordinate 63 of the Z-axis is periodically displaced vertically. The tissue displacement detecting unit 51 monitors the output from the coordinate calculating unit 50, and detects periodical variation of the coordinate position of the probe on the Z-axis as in the case of this embodiment, whereby it is determined that the probe periodically moves, that is, it executes the periodical press/release on the object. Accordingly, it can be automatically detected that the tissue of the cross-sectional site is displaced, so that it can be detected that a proper elasticity image can be acquired.

This embodiment is based on the assumption that the probe is substantially vertically pressed/released against/from the object lying down on his/her bed, and thus the coordinate of the probe periodically varies in only the Z-axis direction. Accordingly, the coordinate varied axis is variously varied in accordance with how to set the three coordinate axes. For example, there may be considered a case where periodical variation appears on any only one of the three coordinate axes, a case where periodical variation appears on any two axes and a case where periodical variation appears on all the three axes. In short, when periodical coordinate variation is detected with respect to any coordinate axis corresponding to at least the displacement direction of the tissue out of the three coordinate axes, it can be detected that a proper elasticity image can be acquired.

### (Second Embodiment of Tissue Displacement Detection)

This embodiment is applicable to both a case where an elasticity image is generated by executing the press/release operation on an object with a probe and a case where an elasticity image is generated by utilizing application of periodical pressure variation to a tissue due to beat or the like of an object.

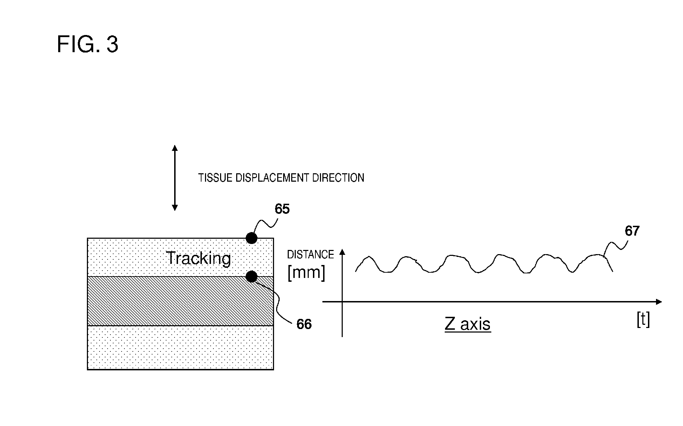

In this embodiment, chasing points 65 and 66 are first set on the generated tomographic image as shown at the left side of Fig. 3. With respect to the setting of the chasing points, an examiner may display a tomographic image on the image display unit 26 and set the chasing points through the interface unit 42, or any plural chasing points on the tomographic image may be automatically set.

Subsequently, the chasing points 65 and 66 are tracked according to various methods such as the block matching method by using the displacement measuring unit 30 or the like to determine a destination. The tissue displacement detecting unit 51 measures the displacement of the distance in the direction corresponding to the displacement direction of the tissue between the chasing points 65 and 66. For embodiment, in this embodiment, the tissue is displaced in the vertical direction. In this case, ameasuredvalue 67 of the displacement varies periodically as shown at the right side of Fig. 3. The tissue displacement detecting unit 51 detects this periodical variation, whereby it can detect the displacement of the tissue of the cross-sectional site.

Furthermore, in this embodiment, thetwochasingpoints are set. However, the number of chasing points is not limited to this value, and three or more chasing points may be set. In addition, the displacement of the distance between any two points is measured, and periodical variation of the measured value of the displacement may be detected. Furthermore, for example, the chasing point of one point may be set without using the distance between chasing points, and periodical variation in the direction corresponding to the displacement direction of the tissue at the position of the chasing point may be detected, whereby the displacement of the tissue of the cross-sectional site can be detected.

This embodiment is particularly effective to a tissue in which internal pressure varies such as a heart, or a blood vessel and a case where a stable elasticity image is more easily acquired by using motion of a body. That is, in this case, since no press is applied from the body surface by the probe, the probe position is fixed and thus the probe position does not move. However, if the displacement in the direction corresponding to the displacement direction of the tissue at the chasing point on the tomographic image is periodical, it could be detected that the tissue of the cross-sectional site is displaced, and thus this embodiment is effective.

### (Third Embodiment of Tissue Displacement Detection)

This embodiment is applied to both of a case where an elasticity image is generated by executing the press/release operation on an object with a probe and a case where an elasticity image is generated by utilizing application of periodical pressure variation to a tissue due to beat or the like of an object.

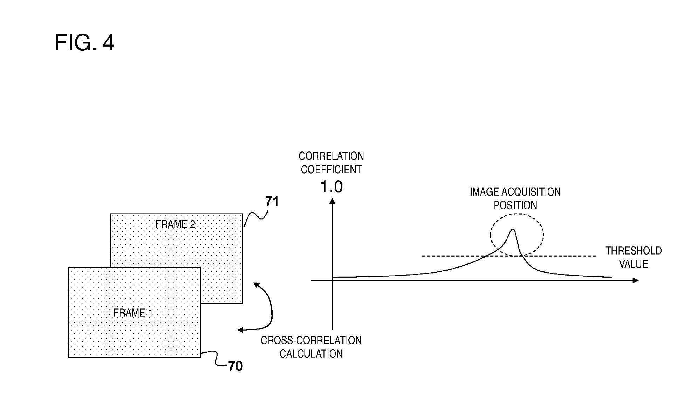

In this embodiment, as shown at the left side of Fig. 4, cross-correlation calculation of a pair of RF signal frame data 70 and 71 selected by the RF frame data selecting unit 28 is executed in the displacement measuring unit 30 functioning as correlation calculating means and then input to the tissue displacement detecting unit. The tissue displacement detecting unit 51 detects the displacement of the tissue of the cross-sectional site on the basis of a calculated correlation coefficient (correlation value). That is, the tissue displacement detecting unit 51 monitors the correlation coefficient input from the correlation calculating means, and detects the displacement of the tissue of the cross-sectional site when the correlation coefficient is larger than a preset threshold value as shown at the right side of Fig. 4.

This embodiment utilizes that when the probe is properly moved to obtain a tomographic image, the correlation coefficient of the pair of RF signal frame data is relatively low, however, when an elasticity image is generated while the press/release operation is executed on the object by using the probe or when an elasticity image is generated by using pressure variation due to beat or the like of the object, the correlation value is relatively high. That is, when a proper press is obtained or a proper displacement can be detected, the correlation degree between the RF signal frame data is enhanced. Therefore, by detecting this, it can be detected that the tissue of the cross-sectional site is displaced and also an elasticity image is acquired.

However, for example when an image of a cross-sectional site at which no variation occurs in the internal pressure of the tissue is picked up and also the probe position is fixed, the pair of RF signal frame data are substantially coincident with each other and the correlation coefficient therebetween indicates a substantially upper limit (for example, 1.0). Accordingly, such a case can be properly excluded from the detection of the tissue displacement.

### (Fourth Embodiment of Tissue Displacement Detection)

This embodiment is applied to both of a case where an elasticity image is generated by executing the press/release operation on an object with a probe and a case where an elasticity image is generated by utilizing application of periodical pressure variation to a tissue due to beat or the like of an object.

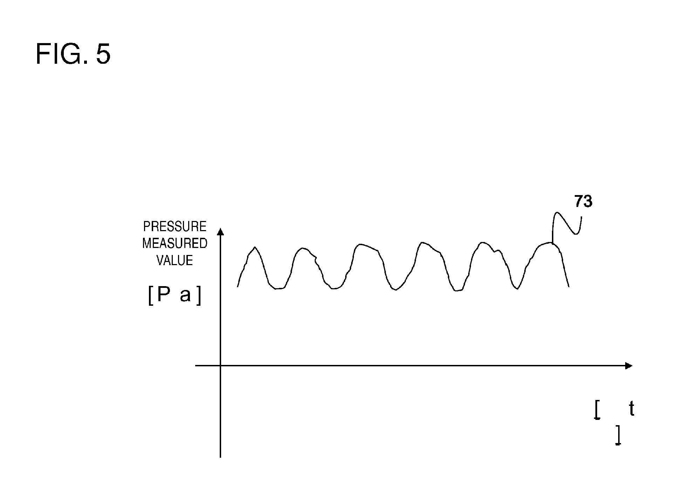

When the pressure measuring unit 46 is provided as in the case of the ultrasonic diagnostic apparatus of this embodiment, the tissue displacement detecting unit 51 takes in the measured value of the pressure measuring unit 46, and detects the displacement of the tissue of the cross-sectional site on the basis of the periodical variation of a measured value 73 of pressure as shown in Fig. 5.

In each of the case where the press/release operation is executed by the probe and the case where the variation of the internal pressure of the object is used, the internal pressure varies when the displacement of the tissue of the cross-sectional site occurs. Therefore, this embodiment utilizes that this internal pressure variation is measured by the pressure measuring unit 46.

### (Fifth Embodiment of Tissue Displacement Detection)

In the above embodiments, the displacement of the tissue is automatically detected by the ultrasonic diagnostic apparatus to detect the acquisition of the elasticity image, however, the present embodiment is not limited to these embodiments. For example, according to a fifth embodiment, an examiner checks the image display unit 26 on a real-time basis while picking up an elasticity image, and manually instructs to acquire an elasticity image through the ultrasonic probe 12, the interface unit 42 or the like. The tissue displacement detecting unit 51 detects the acquisition of an elasticity image on the basis of a signal input through the ultrasonic probe 12 or the interface unit 42.

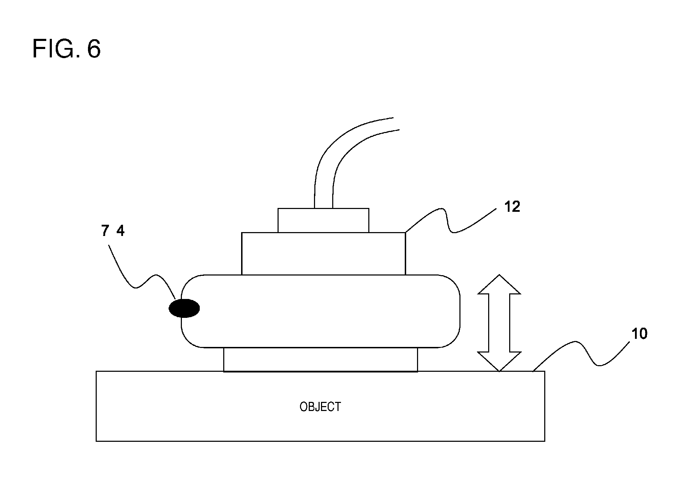

When it is detected on the basis of an instruction of acquiring an elasticity image by the ultrasonic probe 12 that the elasticity image has been detected, a grip portion or the like of the ultrasonic probe 12 is provided with a button-like tissue displacement detecting switch 74 which an examiner can push down as shown in Fig. 6. When the examiner checks the image display unit 26 and determines that a proper elasticity image based on a proper press is generated, the examiner pushes down the tissue displacement detecting switch 74. The tissue displacement detecting unit 51 receives a signal caused by the push-down, and detects that the tissue of the cross-sectional site is displaced and an elasticity image is acquired. The tissue displacement detecting switch is not limited to the button switch, but it may be any member which can be freely operated by the Examiner.

Furthermore, a voice receiving unit such as a microphone may be provided as the interface unit 42, and acquisition of an elasticity image is instructed by using the voice receiving unit, whereby it can be detected that an elasticity image is acquired. That is, a voice signal having a predetermined specific phrase is recorded in the memory in advance, and the tissue displacement detecting unit 51 uses pattern recognition between a voice signal received by the microphone and the recorded voice signal to detect coincidence with or similarity to the recorded voice signal, whereby the displacement of the tissue of the cross-sectional site and the acquisition of a detected elasticity image are detected on the basis of the reception of the corresponding voice signal by the microphone.

In this case, in order to prevent an unnecessary elasticity image from being acquired due to erroneous voice recognition, it is displayed on the image display unit 26 that an elasticity image is acquired through voice recognition, thereby promoting the examiner to determine by voice or a manual operation whether the acquisition is executed or not.

The displacement of the tissue of the cross-sectional site is detected by the tissue displacement detecting unit 51 by any one of the embodiments or properly combining these embodiments. After the displacement of the tissue of the cross-sectional site is detected, the biometric simulation image and the simulation mark, etc. to be superimposed on the biometric simulation image are displayed on the image display unit 26 by the mark generating unit 52 functioning as the display control means, the biometric simulation image constructing unit 54, etc.

An embodiment of a display style of the biometric simulation image and the simulationmark, etc. to be superimposed on the biometric simulation image which are displayed on the image display unit 26 by the mark generating unit 52, the biometric simulation image constructing unit 54, etc. will be described hereunder. In the following description, only the display of the biometric simulation image and the simulation mark, etc. to be superimposed on the biometric simulation image will be described, however, they may be displayed on the image display unit 26 in combination with the tomographic image, the elasticity image, etc.

### (First Embodiment of Display)

In this embodiment, an elasticity image acquiring mark representing a cross-sectional site at which an elasticity image is acquired is superimposedly displayed on a biometric simulation image. In this embodiment and the following embodiments, an image which illustratively simulates a breast of an object is applied as a biometric simulation image.

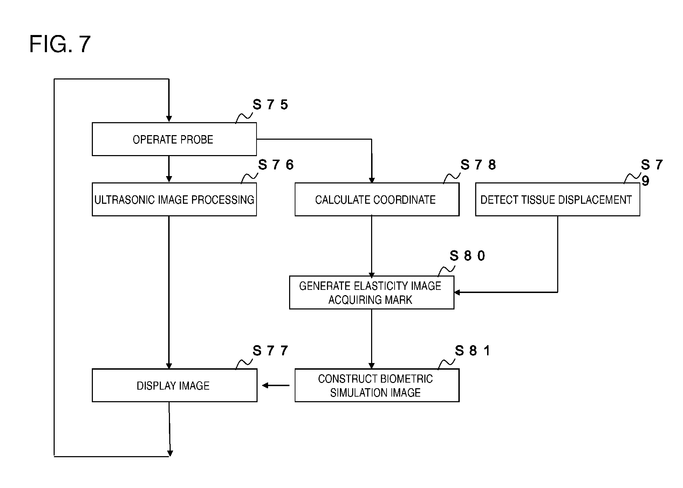

First, the overall operation of the ultrasonic diagnostic apparatus and the operation of the feature portion thereof will be described with reference to Fig. 7. As shown in Fig. 7, in a normal examination routine, ultrasonic waves are transmitted/received while the probe is operated (S75), ultrasonic images such as a tomographic image, and an elasticity image are processed as described above (S76), and these images are displayed (S77).

Furthermore, the coordinate calculation is executed by the coordinate calculating unit 50 (S78), and the position information of a cross-sectional site of the object is obtained. In addition, the variation of a tissue of the cross-sectional site is detected by the tissue displacement detecting unit 51 (S79), and input to the mark generating unit 52. The mark generating unit 52 generates an elasticity image acquiring mark on the basis of an output from the coordinate calculating unit 50 when the displacement of the tissue is detected in the tissue displacement detecting unit 51, that is, on the basis of the position information of the cross-sectional site of the object (S80).

The biometric simulation constructing unit 54 suitably uses the elasticity image acquiring mark generated in the mark generating unit 52, the cross-sectional position information of the object at which the elasticity image stored in the memory 56 is acquired, and information on the output transition of the pressure measuring unit 46 obtained in the process of acquiring the elasticity image, etc. to superimpose the elasticity image acquiring mark at the corresponding position on a biometric simulation image, thereby constructing the biometric simulation image (S81). Accordingly, the ultrasonic image and the biometric simulation image on which the simulation mark is superimposed are properly combined and displayed on the image display unit.

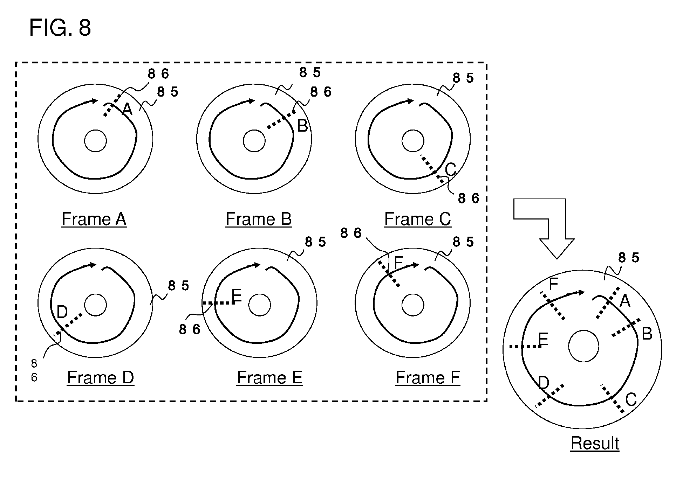

In this embodiment, when the ultrasonic image pickup operation is executed to acquire an elasticity image, an elasticity image acquiring mark 86 representing a cross-sectional site at which the elasticity image is acquired is superimposedly displayed at the corresponding position on a biometric simulation image 85 as shown in Fig. 8. As shown in Fig. 8, the elasticity image acquiring mark 86 contains information on the scan position and direction of the ultrasonic probe.

By displaying the elasticity image acquiring mark 86 on a real-time basis during the ultrasonic image pickup operation, the examiner who executes the ultrasonic image pickup operation checks the biometric simulation image to visually specify a position of the object at which the elasticity image is acquired. Furthermore, when the biometric simulation image 85 on which the elasticity image acquiring mark 86 is superimposed is stored in the memory, a diagnosing person or the like who refers to stored images to make a diagnosis can check the biometric simulation image to visually specify a position of the object at which the elasticity image is acquired.

Furthermore, when the examiner acquires an elasticity image as indicated by Frames A to F of Fig. 8, the elasticity image acquiringmarks 86 may be displayed one by one on a real-time basis, or the mark may be successively superimposedly displayed on the biometric simulation image every time an elasticity image is acquired while the elasticity image acquiring marks 86 which were superimposed on the biometric simulation image in the past are left. According to this manner, sites at which elasticity images of the object have been acquired can be grasped at a glance, so that omission of a desired image can be prevented and needless duplicative image pickup of the same site can be omitted.

The coordinate calculating unit 50 can specify the ultrasonic image pickup position on the biometric simulation image by positioning the coordinate system of the three-dimensional coordinate detected by the position information sensor and the coordinate system of the object while executing the ultrasonic image pickup operation on a specific site such as a breast of the object.

### (Second Embodiment of Display)

In this embodiment, information of pressure or the like measured by the pressure measuring unit 46 in the process of acquiring an elasticity image is displayed through an elasticity image acquiring mark superimposed on a biometric simulation image.

This embodiment is based on the assumption that the elasticity image acquiring mark 86 is superimposed on the biometric simulation image 85, and also the output transition of pressure detected by the pressure measuring unit 46 when an elasticity image corresponding to each elasticity image acquiring mark is acquired is recorded in the memory in association with each elasticity image acquiring mark 86.

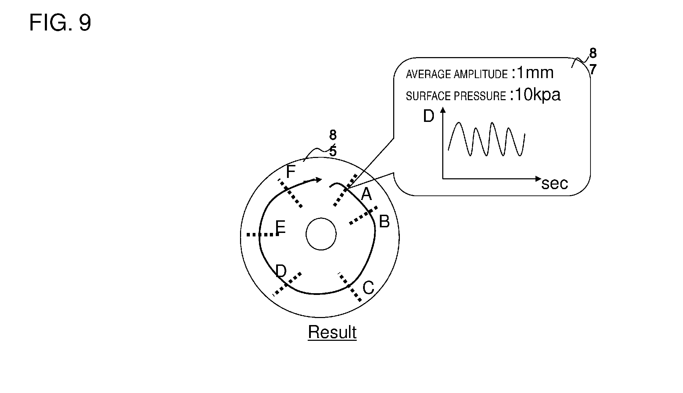

Fig. 9 assumes a state that the examiner checks the biometric simulation image generated in the First Embodiment of Display during the ultrasonic image pickup operation and a state that the diagnosing person or the like checks the biometric simulation image after it is examined and stored in the memory. For example, when the diagnosing person or the like selects any elasticity image acquiring mark through an interface such as a mouse, a keyboard, a track ball, or a screen touch sensor, there is provided a pressure state display 87 containing a graph of the output transition of the pressure sensor associated with the selected mark, the average amplitude of pressure determined from the output transition of the pressure sensor, the average value of pressure determined from the output transition of the pressure sensor, etc.

With respect to the acquisition of the elasticity image, when press is applied by the probe, it is very important whether the press is moderately applied. When the degree of the press is abnormal, it is difficult to acquire a desired elasticity image. Accordingtothisembodiment,whenthe diagnosing person selects an elasticity image acquiring mark of interest while checking a biometric simulation image which is temporarily recorded in the memory, a pressure condition or the like when an elasticity image is acquired at the corresponding cross-sectional site can be displayed. Accordingly, it is possible to operate the ultrasonic probe so that the same pressure condition is obtained while checking the displayed pressure condition or the like. As described above, by multilaterally supplying information to the diagnosing person, the diagnosing person can acquire an elasticity image with excellent reproducibility again in consideration of the displayed pressure condition or the like for a reference. Therefore, this embodiment may be effectively and practically used for diagnosis.

An elasticity image acquiring mark may be selected not only by inputting it from the interface, but also by automatically setting it so that the elasticity image acquiring mark is switched every set time. Furthermore, in addition to the information described in this embodiment, information obtained in the process of acquiring the elasticity image may be properly stored in the memory, and displayed in accordance with selection of the mark. Furthermore, plural elasticity image acquiring marks may be selected, and plural detailed information may be displayed, or a graph, etc. may be superimposedly displayed.

### (Third Embodiment of Display)

In this embodiment, an elasticity mark representing hardness or softness of an area set at a cross-sectional site at which an elasticity image is acquired, that is, a tissue of interest (tumor) is displayed so as to be superimposed on a biometric simulation image.

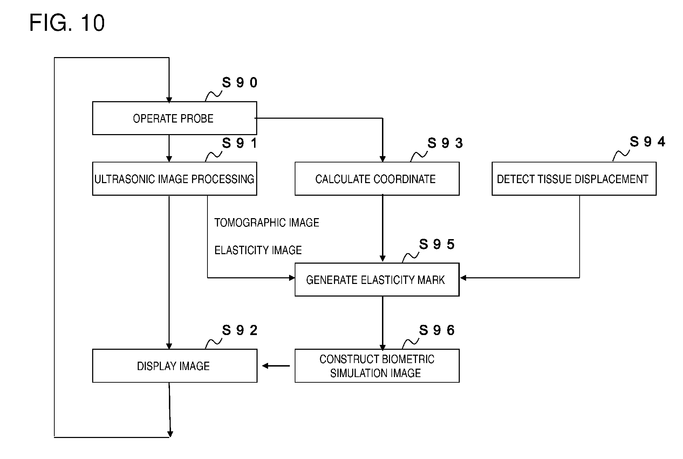

First, the overall operation of the ultrasonic diagnostic apparatus and the operation of the feature portion will be described with reference to Fig. 10. As shown in Fig. 10, in a normal examination routine, ultrasonic waves are transmitted/received while the ultrasonic probe is operated (S90), ultrasonic images such as a tomographic image, and an elasticity image are processed as described above (S91) and these images are displayed (S92).

Furthermore, the coordinate calculation is executed by the coordinate calculating unit 50 (S93), and the position information of a cross-sectional site of the object is obtained. In addition, the variation of a tissue of the cross-sectional site is detected by the tissue displacement detecting unit 51 (S94), and input to the mark generating unit 52. Furthermore, the information of the tomographic image and the elasticity image obtained by the ultrasonic image pickup operation are input to the mark generating unit 52. The mark generating unit 52 generates a simulation mark such as an elasticity mark on the basis of an output from the coordinate calculating unit 50 when the displacement of the tissue is detected in the tissue displacement detecting unit 51, that is, the position information of the cross-sectional site of the object, and the elasticity information representing hardness or softness of a tissue of interest of an area set in the elasticity image (S95).

The biometric simulation image constructing unit 54 arbitrarily uses the elasticity mark generated in the mark generating unit 52 and the information such as the cross-sectional position information of the object at which the elasticity image stored in the memory 56 is acquired, the elasticity information of the tissue in the set area of the elasticity image, and the output transition of the pressure measuring unit 46 obtained in the process of acquiring the elasticity image to superimpose the simulation mark at the corresponding position on a biometric simulation image, thereby constructing the biometric simulation image (S96). Accordingly, the ultrasonic image and the biometric simulation image on which the simulation mark is superimposed are arbitrarily combined and displayed on the image display unit.

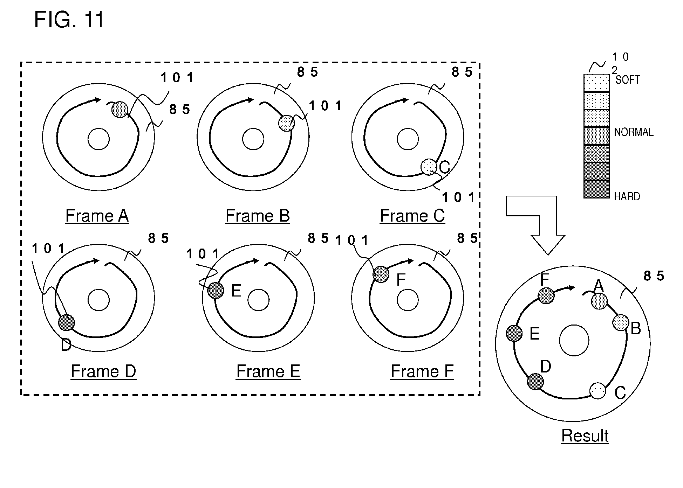

Accordingly, a display shown in Fig. 11 is provided. That is, when an elasticity image is acquired, a circular elasticity mark 101 which is gradated in accordance with hardness or softness of the tissue (tumor) in the set area of the elasticity image is superimposedly displayed at the corresponding position on the biometric simulation image 85. Furthermore, in addition to the circular elasticity mark 101, an elasticity scale bar 102 representing gradated hue and the degree of hardness or softness of the tissue in association with each other is also displayed.

As in the case of the First Embodiment of Display, when an elasticity image is acquired, the elasticity mark 101 may be displayed one by one on a real-time basis, or the mark may be successively displayed so as to be superimposed on a biometric simulation image every time an elasticity image is acquired while elasticity marks superimposed in the past are left on the biometric simulation image. Furthermore, this embodiment adopts the hue gradation, however, the hardness or softness of the tissue may be gradated and displayed with numerical values or brightness.

Furthermore, in this embodiment, the simulation mark is superimposedly displayed on the biometric simulation image. However, the simulation mark may be displayed in association with a tomographic image or an elasticity image by superimposing the simulation mark on a part of the tomographic image or the elasticity image.

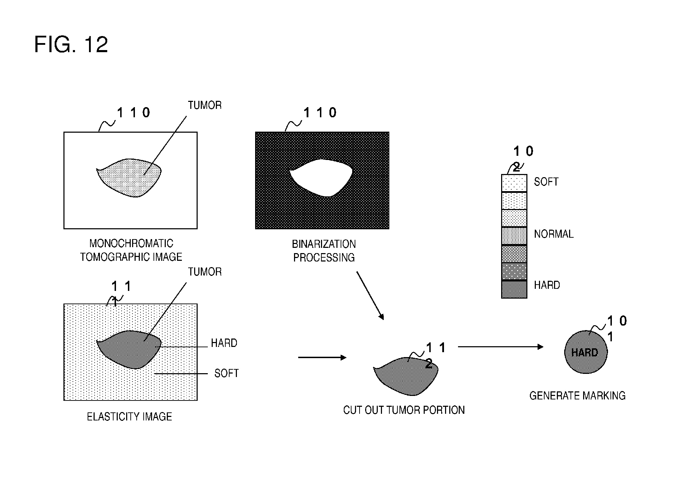

Next, the generation of the elasticity mark 101 will be described in more detail with reference to Fig. 12. The elasticity mark 101 is not the elasticity information of the whole acquired elasticity image, but represents elasticity information of an area set in the elasticity image, that is, a tissue of interest such as a tumor. Therefore, it is necessary to set an area of a tissue of interest from an elasticity image.

In this point, it is utilized that a tissue of interest such as a tumor provides a low echo (low reflection level) on a monochromatic tomographic image 110 as shown in Fig. 12. That is, first, the monochromatic tomographic image is first subjected to binarization processing, and a low-echo area is set as a mask area, thereby detecting the position or distribution of a tumor.

A tissue 112 in the mask area of an elasticity image 111 is cut out, and elasticity information of the cut-out tissue is averaged to generate the elasticity mark 101. The elasticity mark is not required to be circular, and it may be displayed while the shape thereof is also expressed by using distribution information of the tumor.

By making a display as described above, the elasticity information of the tissue of interest in the area set at the cross-sectional site at which the elasticity image is acquired can be simply grasped through the biometric simulation image.

Furthermore, when the examiner executes a series of elasticity-image pickup operation on the object and a biometric simulation image on which an elasticity mark is superimposed is recorded, the diagnosing person can easily determine which site of the object should be examined minutely when the diagnosing person checks the biometric simulation image. That is, the elasticity mark is represented as a simple mark which is gradated, for example, with hue or numerical values, and thus a place where a diseased tissue may exist because it is relatively hard can be grasped at a glance on the biometric simulation image. As occasion demands, the site concerned can be minutely examined by executing the ultrasonic image pickup operation again or the like, thereby making a diagnosis and determining a treatment policy.

### (Fourth Embodiment of Display)

In this embodiment, through an elasticity mark superimposed on a biometric simulation image, an elasticity image which contributes to generation of the elasticity mark is displayed. This embodiment is based on the assumption that elasticity marks 101 are superimposed on the biometric simulation image 85, and also the elasticity images corresponding to the respective elasticity marks are recorded in association with the respective elasticity marks 101 in the memory.

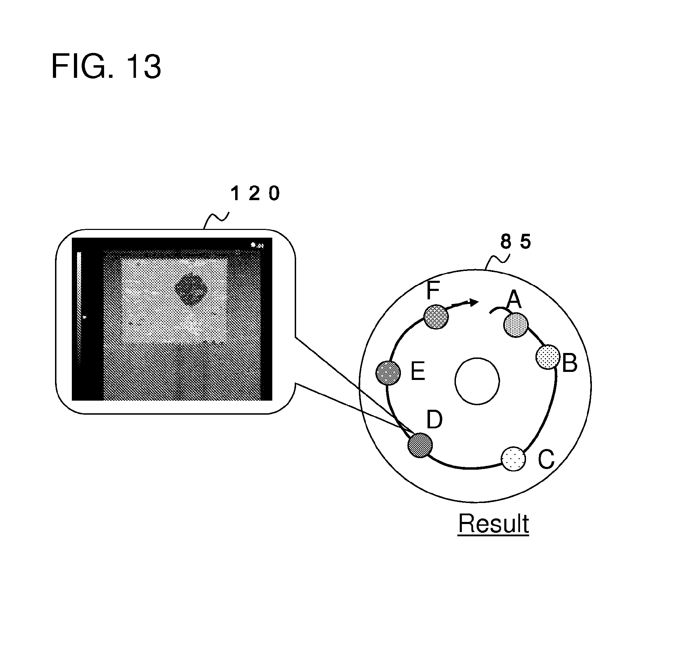

Fig. 13 assumes a state that the examiner checks the biometric simulation image generated in the Third Embodiment of Display during the ultrasonic image pickup operation and a state that the diagnosing person or the like checks the biometric simulation image after it is examined and recorded in the memory. For example, when the diagnosing person or the like selects any elasticity image acquiring mark through an interface such as a mouse, a keyboard, a track ball, or a screen touch sensor, the elasticity image associated with the selected mark is called up from the memory to provide an elasticity image display 120.

According to this embodiment, for example, when the diagnosing person selects an elasticity image acquiring mark of interest while checking a biometric simulation image which is temporarily recorded in the memory and then displayed, the diagnosing person can check an elasticity image at the corresponding cross-sectional place. As described above, by multilaterally supplying information to the diagnosing person, the diagnosing person can make a diagnosis and determine a treatment policy while checking the displayed biometric simulation image and the elasticity marks, determining the whole condition of a tumor of the object and also calling up the elasticity images to check a place of interest minutely.

The call-up of the elasticity image in this embodiment and the call-up of the pressure state described in the second Embodiment of Display may be properly combined and displayed.

### (Fifth Embodiment of Display)

In this embodiment, the elasticity mark is displayed in a gradation style on the biometric simulation image. That is, when an elasticity mark is displayed, there is a case where a target is detected at two or more places. In this case, internal targets may be continuous with each other with high probability. Therefore, the targets can be represented with gradation by using information of the elasticity degree between two points and the distance therebetween.

For example, when the examiner makes an examination and determines that the target is a continuous target (tumor), the area between two points is represented by gradation, whereby continuity can be displayed. Accordingly, the continuity of the target can be reflected to a medical record without repeating press, and thus this embodiment is effective to shorten the examination time.

### (Sixth Embodiment of Display)

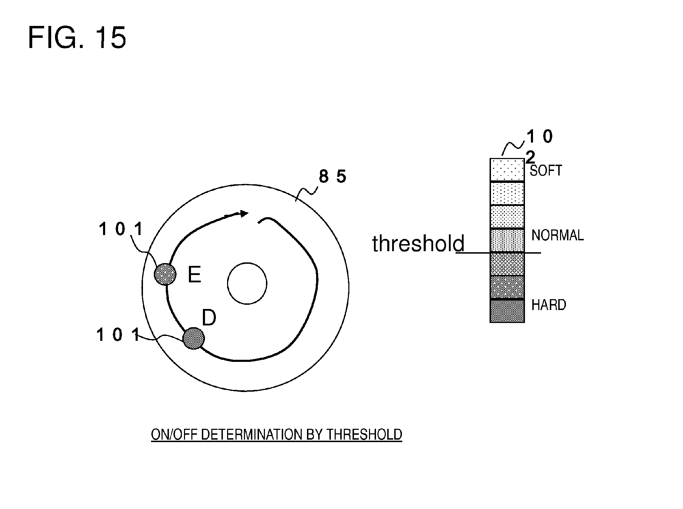

In this embodiment, out of the generated elasticity marks, only gradated elasticity marks whose elasticity information are not less than a preset threshold value, are less than the threshold value or are within a threshold value range are displayed. That is, for example, as shown in Fig. 15, the threshold value is preset by using the elasticity scale bar 102 or the like, or the examiner/the diagnosing person can freely set/adjust the threshold value, whereby only information required by the examiner or the diagnosing person can be displayed on the biometric simulation image. Furthermore, this threshold value may be automatically set every examination site.

Accordingly, out of elasticity marks, only an elasticity mark which is harder than a predetermined threshold value, that is, which possibly represents a lesion and thus requires minute examination can be displayed. Therefore, the examiner/diagnosing person can check only actually necessary information to make a diagnosis, determine a treatment policy, etc. The display of the biometric simulation image and the elasticity marks is performed to simply supply information such as tissue elasticity of an object to an examiner/diagnosing person. Therefore, if the amount of information is excessive, it would not be practical. Accordingly, this embodiment is effective.

### (Seventh Embodiment of Display)

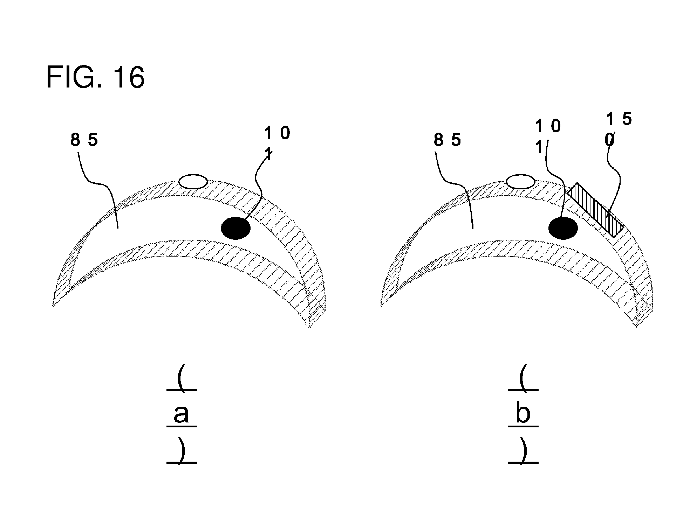

In this embodiment, an elasticity mark is displayed on a three-dimensionally generated biometric insulation image. That is, as shown in Fig. 16(a), the elasticity mark 101 is superimposedly displayed on the three-dimensionally generated biometric insulation image 85 in consideration of position information of a cross-sectional site of an object when an elasticity image is acquired, and depth information from the surface at the cross-sectional site of the object.

Furthermore, as shown in Fig. 16(b), a press mark 150 representing the contact surface position of the ultrasonic probe to the object when the elasticity image is acquired can be superimposedly displayed in addition to the elasticity mark 101.

According to this embodiment, the depth information from an ultrasonic wave transceiver can be left on the medical record as illustration information. For example, in a case where infiltration of cancer is observed in a depth direction which is difficult to be found by using two-dimensional information, it can be three-dimensionally represented as an elasticity mark of a tumor, and thus this embodiment is effective from the viewpoint that it can be easily grasped after examination.

Furthermore, with respect to diagnosis of internal organs, there is a case where it is important for a diagnosis to know which press position brings an image corresponding to an elasticity-mark affixed position. At this time, the press mark 150 is affixed onto the body surface, the elasticity mark 101 is affixed to the inside and both the marks are simultaneously displayed, whereby the press place and the image can be simply associated with each other.

Even in the case of a three-dimensional biometric simulation illustration, when there are many examination positions, it becomes promiscuous, and thus it is concerned that the illustration is difficult to view as a display. Therefore, only the pair of the press position and the tumor position which are selected on the two-dimensional biometric simulation image may be displayed on the three-dimensional biometric simulation image, or coloring, hatching, etc. may be used to represent the combination thereof.

### (Eighth Embodiment of Display)

In this embodiment, in addition to the simulation mark, a blood streammark is superimposedly displayed on the biometric simulation image. This embodiment is based on the assumption that it is provided with blood stream mark generating means for obtaining blood stream information on the basis of a reflection echo signal measured by the ultrasonic probe and generating a blood stream mark gradated in accordance with the blood stream velocity of the cross-sectional site of the object.

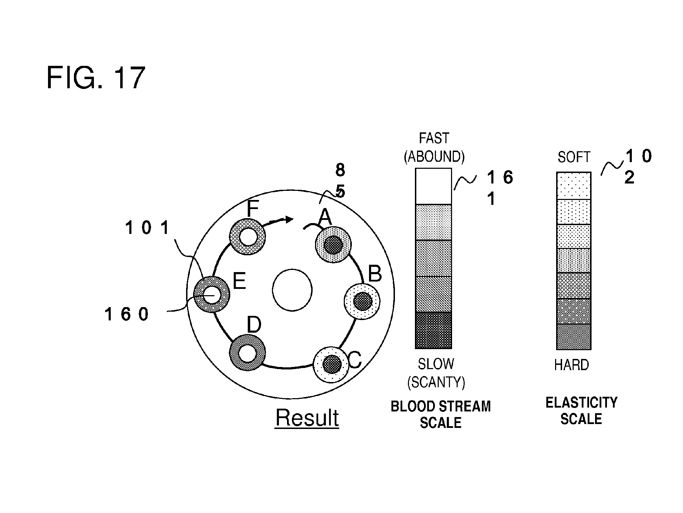

That is, the velocity or abundance of blood stream can be detected by a method using a well-known Doppler effect, and a blood stream mark which is gradated in accordance with the detection result can be generated. As shown in Fig. 17, a blood stream mark 160 may be designed as a smaller circle than the elasticity mark 101, and superimposedly displayed on the elasticity mark 101. In addition, a blood stream scale bar 161 representing the degree of the velocity or abundance of the blood stream may be displayed.

For example, a malignant tumor trends to absorb blood from surrounding tissues and grow. Accordingly, it is expected to contribute to enhancement of diagnosis precision/diagnosis efficiency that blood stream information and elasticity information are simultaneously displayed on a biometric simulation image and used as medical record information.

The blood stream mark 160 and the elasticity mark 101 can be discriminated from each other and easily recognized like the blood stream mark 160 is represented by an asterisk and the elasticity mark 101 is represented by a circle. Furthermore, both the marks can be discriminated from each other and visually enhanced like the blood stream is represented by monochromatic gradation and the elasticity mark is represented by color gradation. Furthermore, only the overlap portion between the blood stream mark and the elasticity mark may be displayed on a biometric illustration.

### (Ninth Embodiment of Display)

In this embodiment, when a re-examination is executed on an object whose elasticity images were acquired by the ultrasonic image pickup operation in the past, an examiner is informed of places at which the elasticity images were acquired in the past (the positions whose elasticity information was recorded).

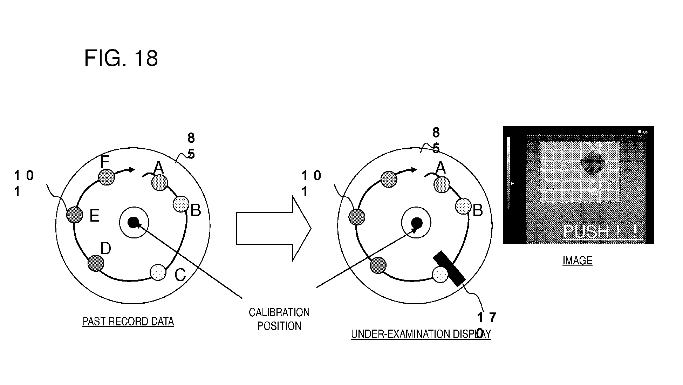

That is, calibration is performed with reference to a specific position such as a mammary papilla of the object to perform position adjustment, and then ultrasonic examination is executed. Then, as shown in Fig. 18, when the ultrasonic probe is moved to a place at which an elasticity image was acquired in the past (when the position of the elasticity mark 101 and a probe mark 170 representing the probe position are overlapped with each other), an indication "PUSH" or the like is displayed on the image display unit 26, whereby the examiner can be promoted to perform the operation of acquiring an elasticity image. At this time, the elasticity images which were acquired in the past may be additionally displayed.

As described above, the place at which an elasticity image should be acquired is presented to the examiner, whereby the labor of the examiner can be reduced and the examination efficiency can be enhanced. At this time, a pressure condition or the like when the elasticity image is acquired is additionally displayed, whereby reproducibility can be enhanced.

The embodiment of the ultrasonic diagnostic apparatus according to the present invention has been described. However, the present invention is not limited to the breast, but may be applied to places which can be subjected to ultrasonic image diagnosis such as thyroid gland, liver, or prostate gland.

## Claims

1. An ultrasonic diagnostic apparatus comprising: an ultrasonic probe that transmits/receives an ultrasonic wave from/to an object; phasing and summing means that generates RF signal frame data of a cross-sectional site of the object on the basis of a reflection echo signal measured by the ultrasonic probe; elasticity image constructing means that generates an elasticity image representing hardness or softness of a tissue of the cross-sectional site on the basis of a pair of RF signal frame data acquired at different times; cross-sectional site information generating means that generates cross-sectional site information containing position information of the cross-sectional site at which the elasticity image is measured; and display control means that controls to display on a display unit the elasticity image generated by the elasticity image constructing means and the cross-sectional site information generated by the cross-sectional site information generating means while the elasticity image and the cross-sectional site information are associated with each other.

2. The ultrasonic diagnostic apparatus according to claim 1, **characterized in that** the cross-sectional site information contains gradation information based on hardness or softness of a tissue.

3. The ultrasonic diagnostic apparatus according to claim 1, further comprising biometric simulation image generating means for generating a biometric simulation image which simulates the object, wherein the display control means displays the biometric simulation image while superimposing the cross-sectional site information on the biometric simulation image.

4. The ultrasonic diagnostic apparatus according to claim 1, wherein
the cross-sectional site information is at least one simulation mark of an elasticity image acquiring mark representing a cross-sectional site at which the elasticity image is acquired, and an elasticity mark which is gradated in accordance with hardness or softness of a tissue in a set area of the acquired elasticity image.

5. The ultrasonic diagnostic apparatus according to claim 3, wherein
the cross-sectional site information is at least one simulation mark of an elasticity image acquiring mark representing a cross-sectional site at which the elasticity image is acquired, and an elasticity mark which is gradated in accordance with hardness or softness of a tissue in a set area of the acquired elasticity image, and the display control means displays on a display unit the simulation mark while superimposing the simulation mark on a cross-sectional site on the biometric simulation image at which the elasticity image is acquired.

6. The ultrasonic diagnostic apparatus according to claim 5, wherein the display control means successively displays a simulation mark while superimposing the simulation mark on the biometric simulation image every time the elasticity image is acquired while simulation marks superimposed in the past are left on the biometric simulation image.

7. The ultrasonic diagnostic apparatus according to claim 5, wherein an elasticity image corresponding to a simulation mark superimposed on the biometric simulation image is recorded in a memory in association with the simulation mark, and when the simulation mark is selected through an interface, the display control means displays the elasticity image associated with the selected simulation mark.

8. The ultrasonic diagnostic apparatus according to claim 5, wherein when an elasticity image corresponding to a simulation mark superimposed on the biometric simulation image is acquired, an output transition detected by a pressure sensor provided to a contact face of the ultrasonic probe to the object is recorded in a memory in association with the simulation mark concerned, and when the simulation mark is selected through an interface, the display control means displays at least one of the output transition of the pressure sensor associated with the selected simulation mark, an average amplitude of pressure determined from the output transition of the pressure sensor and an average value of pressure determined from the output transition of the pressure sensor.

9. The ultrasonic diagnostic apparatus according to claim 5, wherein the display control means displays only elasticity marks gradated under a condition that the elasticity marks are not less than a preset threshold value, less than the threshold value or within a threshold value range, out of the elasticity marks which are gradated in accordance with hardness or softness of the tissue in the set area of the acquired elasticity image.

10. The ultrasonic diagnostic apparatus according to claim 5, wherein the biometric simulation image generating means generates a three-dimensional biometric simulation image, and the display control means superimposedly displays the elasticity mark on the three-dimensional biometric simulation image on the basis of position information of a cross-sectional site of the object when the elasticity image is acquired and depth information from the surface at the cross-sectional site of the object.

11. The ultrasonic diagnostic apparatus according to claim 5, further comprising blood stream mark generating means for obtaining blood stream information on the basis of the reflection echo signal measured by the ultrasonic probe and generating a blood stream mark which is gradated in accordance with a blood stream velocity of the cross-sectional site of the object, wherein the display control means superimposedly displays the blood stream mark on the biometric simulation image in addition to the simulation mark.

12. The ultrasonic diagnostic apparatus according to claim 5, further comprising a position sensor that is mounted on the ultrasonic probe and detects a three-dimensional coordinate position and an attitude of the probe, and cross-sectional site detecting means for detecting position information of a cross-sectional site of the object on the basis of an output from the position sensor, wherein the elasticity image acquiring mark is generated on the basis of the position information of the cross-sectional site of the object detected by the cross-sectional site detecting means when the elasticity image is acquired, and the elasticity mark is generated in accordance with the position information of the cross-sectional site of the object detected by the cross-sectional site detecting means when the elasticity image is acquired, and hardness or softness of a tissue in a mask area set in an area in which the intensity of the RF signal of the elasticity image is smaller than a preset threshold value.
